# EUROPEAN PATENT SPECIFICATION

(11) **EP 2 661 271 B1**
(45) Date of publication and mention of the grant of the patent: **08.07.2020**
(21) Application number: 12732443.2
(22) Date of filing: 05.01.2012
(51) Int. Cl.: A61K 31/727, A61K 31/167, A61P 13/10

(54) **METHOD FOR MANUFACTURING COMPOSITION COMPRISING LOCAL ANESTHETIC, HEPARINOID, AND BUFFER**
VERFAHREN ZUR HERSTELLUNG EINER ZUSAMMENSETZUNG MIT EINEM LOKALANÄSTHETIKUM, EINEM HEPARINOID UND EINEM PUFFER
PROCÉDÉ DE FABRICATION D'UNE COMPOSITION COMPRENANT UN ANESTHÉSIQUE LOCAL, UN HÉPARINOÏDE, ET UN TAMPON

(30) Priority: 06.01.2011 US 201161430468 P
(43) Date of publication of application: 13.11.2013
(73) Proprietor: Parsons, C. Lowell, Henderson, Nevada 89011 (US)
(72) Inventor: Parsons, C. Lowell, Henderson, Nevada 89011 (US)
(74) Representative: Körfer, Thomas
(86) International application number: PCT/US2012/020357
(87) International publication number: WO 2012/094515

(56) References cited:
- WO-A2-2006/076663
- WO-A2-2007/072147
- US-A1- 2005 234 013
- US-A1- 2010 028 438
- PARSONS C L: "Evidence-based strategies for recognizing and managing IC", CONTEMPORARY UROLOGY, ADVANSTAR COMMUNICATIONS, US, 1 February 2003 (2003-02-01), pages 22-35, XP008097855, ISSN: 1042-2250

## Description

### FIELD OF THE INVENTION

This invention is directed to an improved method of manufacturing a composition suitable for the treatment of urinary bladder symptoms as seen in interstitial cystitis and other lower urinary tract conditions such as, but not limited to, overactive bladder, prostatitis, urethral syndrome in women, and gynecologic chronic pelvic pain. The composition comprises a local anesthetic, typically lidocaine, a glycosaminoglycan, typically heparin, and a buffer, typically sodium bicarbonate which is then instilled directly into the urinary bladder.

### BACKGROUND OF THE INVENTION

Interstitial cystitis (IC) is a chronic progressive disorder of the lower urinary tract that causes urinary urgency and frequency and/or pelvic pain. For many years, urologists regarded IC as a rare disease for which they had no broadly effective treatment. In fact, the condition is quite common. In 1999, prevalence in the United States was estimated at 750,000 cases (Curhan, et al. J Urol 161(2):549-552 (1999)). However the true prevalence of IC is estimated to be at least 1-2 million patients who are suffering from severe chronic pelvic pain. In addition overactive bladder, urethral syndrome, prostatitis, and gynecologic chronic pelvic pain syndrome comprises millions of patients that also result in bladder symptoms of urgency, frequency, incontinence and or pelvic pain with no effective therapy and all these syndromes share similar symptoms and likely a common pathophysiology with traditionally diagnosed IC (Parsons, CL Int Br J Urol Dec, 2010); there are no broadly effective treatments for these conditions.

Therefore, treatments that would both benefit a larger portion of the patient population, provide immediate relief of symptoms without causing additional pain, without requiring extensive alterations in diet, and further provide reversal of the disease process over time are necessary.

Compositions and methods for the treatment of interstitial cystitis are described in United States Patent No. 7,414,039 to Parsons, issued August 19, 2008 and entitled "Interstitial Therapy for Immediate Symptom Relief and Chronic Therapy in Interstitial Cystitis," and in United States Patent Application Publication No. 2008/0300219 by Parsons, published December 4, 2008 and entitled "Novel Interstitial Therapy for Immediate Symptom Relief and Chronic Therapy in Interstitial Cystitis, as well as PCT Patent Application Publication No. WO 2006/076663 A2 by Flashner et al., published July 20, 2006 and entitled "Kits and Improved Compositions for Treating Lower Urinary Tract Disorders," and PCT Patent Application Publication No. WO 2007/073397 by Flashner et al., published June 28, 2007 and entitled Kits and Improved Compositions for Treating Lower Urinary Tract Disorders,".

In general, the compositions disclosed in this issued patent and these published patent applications comprise a local anesthetic, typically lidocaine, a heparinoid, typically heparin, and a buffer, typically sodium bicarbonate that are instilled directly into the urinary bladder. Further, publication no. WO 2007/072147 A2 by Ernst et al., published on June 28, 2007 and entitled "Composition for Diagnosing and Treating Circulatory System Diseases" discloses the composition by injecting into the blood vessel. It fails to mention a composition for the treatment of urinary tract condition by instilling the composition into the unitary bladder or the preparation thereof. The publication entitled "Evidence-based Strategies for Recognizing and Managing IC" by Parsons in Contemporary Urology published in February 2003 describes a buffered solution comprising heparin and lidocaine for intravesical administration but does to mention the method for preparing the same or the advantageous effects associated therein. The Compositions for treatment of urinary tract condition is described in Patent Application No. US 2005/0234013 A1 by Parsons published on October 20, 2005 and entitled "Novel Interstitial Therapy for Immediate Symptom Relief and Chronic Therapy in Interstitial Cystitis". In this document, a heparinoid and a local anesthetic in buffer solution are suggested, however, does not disclose a method for preparing those compositions or details of the stability or lack of stability of the compositions. Disadvantageously fails to suggest how the order of addition of the component or the provision of any of the components either in solid or liquid form would affect the stability of the composition.

Alkalinized lidocaine and heparin can be used to successfully treat bladder symptoms such as, but not limited to, urinary frequency, urgency, incontinence and bladder generated pain. Pain generated by the urinary bladder (a visceral organ) is not always perceived to be arising from the bladder. Pain can be referred anywhere from the navel to the knees and will also refer from the lumbar area down the buttocks to the legs and often has no relation to bladder filling or emptying. Consequently the origin of pelvic pain may not be recognized to be from the bladder. These bladder symptoms can be seen in a variety of "clinical syndromes" which may actually be all from one disease process: a dysfunctional epithelium (Parsons, CL Int Br J Urol, Dec 2010)). Nonetheless all these syndromes that can generate bladder symptoms that can be successfully treated with this solution, including, but not limited to, overactive bladder, interstitial cystitis, urethral syndrome in women, recurrent lower urinary tract infection, prostatitis (male chronic pelvic pain syndrome), radiation cystitis, chemical cystitis, gynecologic chronic pelvic pain syndrome (e.g. endometriosis, vulvodynia, vulvovaginitis, yeast vaginitis).

However, there is a problem in mixing these three compounds, as the wrong balance will result in the precipitation of lidocaine and loss of efficacy. Lidocaine when exposed to pH's at or above 7.0 will de-ionize and absorb through lipid membranes such as the bladder epithelium. As a result, the absorbed lidocaine will anesthetize the bladder nerves and relieve bladder symptoms noted above. The heparin will "coat" the bladder wall and inhibit the diffusion of potassium that is provoking the bladder symptoms in the first place. So the combination provides prolonged relief of bladder symptoms (Parsons, Urology 2003). However, mixing the heparin, lidocaine, and buffering agent has to be done in an exact way to prevent the precipitation of the lidocaine since lidocaine will precipitate at pH values above 7 depending on the conditions. The precipitation of lidocaine reduces its bioavailability and reduces the efficacy of the composition.

Accordingly, there is a need for an improved method of preparation for compositions including a heparinoid such as heparin, a local anesthetic such as lidocaine, and a buffer such as sodium bicarbonate, that will avoid precipitation of the local anesthetic such as lidocaine and thus will maintain the bioavailability of the local anesthetic and maintain the efficacy of the composition. In all instances the preparation of these mixtures is for instillation directly into the urinary bladder for the treatment of bladder symptoms. As such the osmolality, specific cation concentrations and pH requirements are not as restrictive as for the preparations of these drugs for intravenous of subcutaneous administration.

### SUMMARY OF THE INVENTION

An improved method of preparation of a composition including a heparinoid such as heparin, a local anesthetic such as lidocaine, and a buffer such as sodium bicarbonate, prevents precipitation of the local anesthetic such as lidocaine and thus maintains the bioavailability of the local anesthetic. This maintains the stability and efficacy of the composition.

One aspect of the invention is a method for preparing a composition useful for treatment of a lower urinary tract condition by instilling the composition into the urinary bladder, the composition comprising a mixture comprising a heparinoid, a local anesthetic, and a buffer, the method comprising the steps of:
(1) providing a heparinoid in solid form;
(2) providing a local anesthetic in solid form;
(3) dissolving the heparinoid in solid form and the locan anesthetic in solid form in water;
(4) adding a liquid buffer to the mixture of the heparinoid and the local anesthetic dissolved in water to form a mixture of the three components; and
(5) adjusting the pH of the mixture of the liquid buffer, the local anesthetic, and the heparinoid so that a pH is achieved of from 6.7 to 8.3 is achieved without precipitation of the local anesthetic. An optimal pH is 7.4.

Typically, the heparinoid is selected from the group consisting of heparin, chondroitin sulfate, heparan sulfate, hyaluronic acid, keratan sulfate, dermatan sulfate, hyaluronan, and sodium pentosanpolysulfate. Preferably, the heparin is selected from the group consisting of heparin and sodium pentosanpolysulfate. More preferably, the heparinoid is heparin, such as heparin sodium.

Typically, the local anesthetic is selected from the group consisting of benzocaine, lidocaine, tetracaine, bupivacaine, cocaine, etidocaine, flecainide, mepivacaine, pramoxine, prilocaine, procaine, chloroprocaine, oxyprocaine, proparacaine, ropivacaine, dyclonine, dibucaine, propoxycaine, chloroxylenol, cinchocaine, dexivacaine, diamocaine, hexylcaine, levobupivacaine, propoxycaine, pyrrocaine, risocaine, rodocaine, and pharmaceutically acceptable derivatives and bioisosteres thereof, and a combination thereof. Preferably, the local anesthetic is selected from the group consisting of lidocaine, bupivacaine, benzocaine, tetracaine, etidocaine, flecainide, prilocaine, and dibucaine, and a combination thereof. More preferably, the local anesthetic is lidocaine, such as lidocaine hydrochloride.

Typically, the buffer is selected from the group consisting of bicarbonate buffer, Tris (Tris(hydroxymethyl)aminomethane) buffer, MOPS buffer (3-(N-morpholino)propanesulfonic acid), HEPES (N-(2-hydroxyethyl)piperazine-N-(2-ethanesulfonic acid) buffer, ACES (2-[(2-amino-2-oxoethyl)amino]ethanesulfonic acid) buffer, ADA (N-(2-acetamido)2-iminodiacetic acid) buffer, AMPSO (3-[(1,1-dimethyl-2-hydroxyethyl)amino]-2-propanesulfonic acid) buffer, BES (N,N-bis(2-hydroxyethyl)-2-aminoethanesulfonic acid buffer, Bicine (N,N-bis(2-hydroxyethylglycine) buffer, Bis-Tris (bis-(2-hydroxyethyl)imino-tris(hydroxymethyl)methane buffer, CAPS (3-(cyclohexylamino)-1-propanesulfonic acid) buffer, CAPSO (3-(cyclohexylamino)-2-hydroxy-1-propanesulfonic acid) buffer, CHES (2-(N-cyclohexylamino)ethanesulfonic acid) buffer, DIPSO (3-[N,N-bis(2-hydroxyethyl)amino]-2-hydroxy-propanesulfonic acid) buffer, HEPPS (N-(2-hydroxyethylpiperazine)-N'-(3-propanesulfonic acid) buffer, HEPPSO (N-(2-hydroxyethyl)piperazine-N'-(2-hydroxypropanesulfonic acid) buffer, MES (2-(N-morpholino)ethanesulfonic acid) buffer, triethanolamine buffer, imidazole buffer, glycine buffer, ethanolamine buffer, phosphate buffer, MOPSO (3-(N-morpholino)-2-hydroxypropanesulfonic acid) buffer, PIPES (piperazine-N,N'-bis(2-ethanesulfonic acid) buffer, POPSO (piperazine-N,N'-bis(2-hydroxypropaneulfonic acid) buffer, TAPS (N-tris[hydroxymethyl)methyl-3-aminopropanesulfonic acid) buffer; TAPSO (3-[N-tris(hydroxymethyl)methylamino]-2-hydroxy-propanesulfonic acid) buffer, TES (N-tris(hydroxymethyl)methyl-2-aminoethanesulfonic acid) buffer, tricine (N-tris(hydroxymethyl)methylglycine buffer), 2-amino-2-methyl-1,3-propanediol buffer, 2-amino-2-methyl-1-propanol buffer, and a combination thereof. Preferably, the buffer is selected from the group consisting of bicarbonate buffer, Tris buffer, and a combination thereof. More preferably, the buffer is bicarbonate buffer, such as sodium bicarbonate.

The method can further comprise the step of adding one or more of: (i) an osmolar component; (ii) a compound that enables persistence of the composition to the surface of the bladder epithelium; (iii) an antibacterial agent; (iv) an antifungal agent; (v) a vasoconstrictor; or (vi) a preservative, subsequent to preparation of a buffered composition including the heparinoid, the local anesthetic, and the buffer.

Both the heparinoid and the local anesthetic are provided in solid form; the solid form can be a powdered form.

Preferably, the heparinoid is heparin, the local anesthetic is lidocaine, and the buffer is bicarbonate buffer. More preferably, the heparin is heparin sodium, the local anesthetic is lidocaine hydrochloride, and the bicarbonate buffer is sodium bicarbonate.

The composition can be suitable for treating, ameliorating, or preventing a lower urinary tract disorder including, but not limited to, a lower urinary tract disorder selected from the group consisting of bacterial cystitis, fungal/yeast cystitis, vulvar vestibulitis, vulvodynia, dyspareunia, urethral syndrome, and endometriosis in women; prostatitis and chronic pelvic pain syndrome in men; and radiation-induced cystitis, chemotherapy-induced cystitis, interstitial cystitis, and overactive bladder in men or women. Typically, the lower urinary tract disorder is interstitial cystitis.

### DETAILED DESCRIPTION OF THE INVENTION

An improved method of preparation of a composition including a heparinoid such as heparin, a local anesthetic such as lidocaine, and a buffer such as sodium bicarbonate comprises the following steps: steps, as laid out in claim 1:
(1) The first step is obtaining a heparinoid in solid form, such as in a powdered form, such as heparin or sodium pentosanpolysulfate containing no other agents such as an appropriate cation (i.e., sodium) and a local anesthetic such as lidocaine hydrochloride in solid form, such as a powdered form. Buffer (powdered or liquid) should not be added to the local anesthetic in solid form at this stage as it will result in precipitation of the lidocaine when the mixture is hydrated.
(3) Once a composition including both heparinoid and local anesthetic is prepared, the composition, which is a solution at this point, is then buffered by gradually adding the buffering agent, such as sodium bicarbonate, until a pH of from 6.7 to 8.3 or possibly higher is achieved without precipitation of the local anesthetic. Preferably, the pH of the resulting composition is from 7.0 to 7.8. More preferably, the pH of the resulting composition is about 7.4. The amount of the buffering agent required at this stage may vary with each preparation, so the pH should be continually monitored to ensure that the quantity of buffer added is appropriate.
(4) Lastly, a preservative can be added as necessary; this does not result in precipitation.
(6) A final liquid product that has been produced by a method such as those described above will be stable and can be stored for three months or more with no precipitation. Accordingly, a premixed liquid composition prepared according to the present invention that is stable for at least three months with no precipitation of the lidocaine is within the scope of the present invention.

It is necessary to begin the preparation of the composition with powdered heparinoid (heparin) and powdered local anesthetic, as commercially available heparin and lidocaine solutions that are sold in the United States are intended for systemic injection into the body. These are the only products available should someone want to compound them for use into the bladder. These available heparin and lidocaine solutions are not compatible when the buffer is added; the lidocaine precipitates regardless of subsequent attempts to avoid precipitation and maintain the lidocaine in solution.

The composition can further comprise an osmolar component as described further below.

As used herein, "heparinoid" refers to any molecule comprising a glycosaminoglycan which refers to a molecule comprising a network of long, branched chains of sugars (e.g., chondroitin sulfate, heparan sulfate, hyaluronic acid, keratan sulfate, dermatan sulfate, hyaluronan, sodium pentosanpolysulfate, and the like) and optimally further comprising smaller, nitrogen-containing molecules (e.g. low molecular weight molecules). It is not meant to limit the present invention to any one glycosaminoglycan (GAG) or source of GAG. GAG molecules include but are not limited to low molecular weight (LMW) GAGs, naturally derived GAGs, biotechnologically prepared GAGs, chemically modified GAGs, synthetic GAGs, and the like. Heparinoids can also be comprised of pentoses instead of hexoses (GAGs are comprised of hexoses) such as pentosanpolysulfate. It is not meant to limit the present invention to any one heparinoid molecule or source of heparinoid molecule. As used herein, "heparin" refers to a heterogeneous group of straight-chain anionic glycosaminoglycans, as described above, having anticoagulant properties with a molecular weight ranging from 2,000 to 40,000 Da. Heparin is measured by its specific anticoagulation activity in units. In some embodiments, heparin is a higher molecular weight species ranging from 8,000-40,000 daltons. As used herein, "low-molecular-weight heparins" refers to a lower molecular weight (LMW) species ranging from 2,000-8,000 daltons. Sodium pentosanpolysulfate can range from 4,000-6,000 daltons. Also included within the scope of the invention are polymers such as dalteparin or enoxaparin. LMW heparins are made by enzymatic or chemical controlled hydrolysis of unfractionated heparin and have very similar chemical structure as unfractionated heparin except for some changes that may have been introduced due to the enzymatic or chemical treatment. While not intending to limit the mechanism of action of the invention's compositions, the mechanism of action of these drugs may be similar to that of full-length heparin. LMW heparins are usually isolated from bulk heparin. In one embodiment, heparin or another heparinoid is a heparin salt. As used herein, the phrases "pharmaceutically acceptable salts", "a pharmaceutically acceptable salt thereof" or "pharmaceutically accepted complex" for the purposes of this application are equivalent and refer to derivatives prepared from pharmaceutically acceptable non-toxic acids or bases including inorganic acids and bases and organic acids and bases.

Because of the negative charges of these polysaccharides due to the occurrence of sulfate groups and/or carboxylic acid groups in them, they are administered in the form of salts, with an appropriate cation to neutralize the negative charges on the acid groups. Typically, the cation is sodium. However, other physiologically tolerable counterions that do not induce urinary tract dysfunctions, such as magnesium, aluminum, calcium, ammonium, or salts made from physiologically acceptable organic bases such as, but not limited to, trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, dibenzylpiperidine, N-benzyl-*p*-phenethylamine, dehydroabietylamine, N,N'-bisdehydroabietylamine, glucamine, N-methylglucamine, collidine, quinine, quinoline, and basic amino acids such as lysine and arginine, can be used. These cationic counterions can alternatively be used as the counterions with anionic buffers such as bicarbonate, as well. Sodium is typically employed as the positively-charged counterion as indicated above. These salts may be prepared by methods known to those skilled in the art. However, it is generally undesirable to use potassium as a counterion due to its role in the etiology of the conditions and syndromes being treated. Other polysaccharides that have the required activity include, but are not limited, to dextran sulfate and carrageenan. Other glycosaminoglycans can be used in methods according to the invention, including low molecular weight (LMW) glycosaminoglycans, naturally derived glycosaminoglycans, biotechnologically prepared glycosaminoglycans, chemically modified glycosaminoglycans, and synthetic glycosaminoglycans and linear anionic polysaccharides comprised of pentoses.

In some embodiments, a heparinoid comprises a heparin-like molecule (e.g. heparan sulfate). For example, a heparin-like molecule such as heparan sulfate is a glycocosaminoglycans with a structure similar to heparin with the difference being that heparan sulfate has undergone less polymerization than heparin and so has more glucuronic acid and N-acetyl glucosamine than heparin. Heparan sulfate contains fewer sulfate groups, so is not as effective as an anticoagulant as heparin. Heparin exists in a variety of forms characterized by different degrees of sulfation. Typically, heparin has a molecular weight of from about 2 kDa to about 40 kDa. Heparin and heparan sulfate are both characterized by repeating units of disaccharides containing a uronic acid (glucuronic or iduronic acid) and glucosamine, which is either N-sulfated or N-acetylated. The sugar residues may be further O-sulfated at the C-6 and C-3 positions of the glucosamine and the C-2 position of the uronic acid. There are at least 32 potential unique disaccharide units in this class of compounds. Five examples of sugars occurring in heparin are: (1) α-L-iduronic acid 2-sulfate; (2) 2-deoxy-2-sulfamino-α-D-glucose 6-sulfate; (3) β-D-glucuronic acid; (4) 2-acetamido-2-deoxy-α-D-glucose; and (5) α-L-iduronic acid.

In one embodiment, heparin contains at least 130 USP units per mg. Heparin is measured by its specific anticoagulation activity in units; either USP units or international units (IU) are specified in stating the activity of heparin. As used herein, "USP unit" refers to the quantity of heparin that prevents 1.0 ml of citrated sheep plasma from clotting for 1 hour after the addition of 0.2 ml of 1% CaCl₂ at 20° C when compared to a USP reference standard (defined as units/ml). As used herein, "IU" refers to the quantity of heparin that is active in assays as established by the Fifth International standard for Unfractionated Heparin (WHO-5) (defined as International Units/ml) (Linhardt, R. J. & Gunay, N. S. (1999) Semin Thromb Hemost 25, 5-16.).

Particularly preferred heparinoids for use in methods according to the present invention and compositions prepared by those methods include heparin and sodium pentosanpolysulfate. A most particularly preferred heparinoid for use in methods according to the present invention and compositions prepared by those methods is heparin. A preferred form of heparin is heparin sodium, although, as described above, other counterions can be used. The quantity of heparin in compositions prepared according to methods of the present invention can range up to as high as from about 200,000 units to about 250,000 units per unit dose of the composition; any intermediate quantity of heparin, such as, but not limited to, 5,000 units, 10,000 units, 15,000 units, 20,000 units, 25,000 units, 30,000 units, 35,000 units, 40,000 units, 45,000 units, 50,000 units, 55,000 units, 60,000 units, 65,000 units, 70,000 units, 75,000 units, 80,000 units, 85,000 units, 90,000 units, 95,000 units, 100,000 units, 110,000 units, 120,000 units, 130,000 units, 140,000 units, 150,000 units, 160,000 units, 170,000 units, 180,000 units, 190,000 units, 210,000 units, 220,000 units, 230,000 units, or 240,000 units per unit dose of the composition can be used.

The quantity of heparinoid in the composition will vary depending on the subject, the severity and course of the disease, the subject's health, the response to treatment, pharmacokinetic considerations such as liver and kidney function, and the judgment of the treating physician. Accordingly, a number of compositions including differing quantities of heparin per unit dose can be prepared by methods according to the present invention.

In accordance with the practice of the invention, merely by way of example, when the heparinoid is sodium pentosanpolysulfate, the amount of heparinoid in the composition may be about 1 mg to about 1500 mg of sodium pentosanpolysulfate per unit dose (for example about 100 mg to about 600 mg per unit dose of sodium pentosanpolysulfate). In accordance with the practice of the invention, merely by way of example, when the heparinoid is heparan sulfate, the amount of heparinoid in the composition may be about 0.5 mg to about 10,000 mg of heparan sulfate per unit dose (for example about 100 mg to about 300 mg per unit dose of heparan sulfate). In accordance with the practice of the invention, merely by way of example, when the heparinoid is hyaluronic acid, the amount of heparinoid in the composition may be about 5 mg to about 1500 mg of hyaluronic acid per unit dose (for example about 10 mg to about 100 mg per unit dose of hyaluronic acid). In accordance with the practice of the invention, merely by way of example, when the heparinoid is chondroitin sulfate, the amount of heparinoid in the composition may be about 1 mg to about 10,000 mg of chondroitin sulfate per unit dose (for example about 100 mg to about 300 mg per unit dose of chondroitin sulfate). In accordance with the practice of the invention, merely by way of example, when the heparinoid is heparin sodium, the amount of heparinoid in the composition may be about 10 mg to about 1000 mg of heparin sodium per unit dose.

The local anesthetic is typically a sodium channel blocker, such as, but not limited to, the drugs referred to commonly as the "caine" drugs, as well as other sodium channel blockers. The local anesthetic in a composition prepared according to the methods of the present invention can be, but is not limited to, any of benzocaine, lidocaine, tetracaine, bupivacaine, cocaine, etidocaine, flecainide, mepivacaine, pramoxine, prilocaine, procaine, chloroprocaine, oxyprocaine, proparacaine, ropivacaine, dyclonine, dibucaine, propoxycaine, chloroxylenol, cinchocaine, dexivacaine, diamocaine, hexylcaine, levobupivacaine, propoxycaine, pyrrocaine, risocaine, rodocaine, and pharmaceutically acceptable derivatives and bioisosteres thereof, or a combination thereof. Preferably, the anesthetic (e.g., local anesthetic) is selected from the group consisting of lidocaine, bupivacaine, benzocaine, tetracaine, etidocaine, flecainide, prilocaine, and dibucaine, or a combination thereof. A particularly preferred local anesthetic is lidocaine, such as lidocaine hydrochloride. As used herein, the recitation of a local anesthetic includes all salts of that local anesthetic that are compatible with the desired pH, the buffer used, and any counterions present; the recitation of a local anesthetic is not intended to limit the salt form or counterion used beyond these criteria.

The quantity of local anesthetic in the composition will vary depending on the subject, the severity and course of the disease, the subject's health, the response to treatment, pharmacokinetic considerations such as liver and kidney function, and the judgment of the treating physician. Accordingly, a number of compositions including differing quantities of local anesthetic per unit dose can be prepared by methods according to the present invention. For example, the amount of anesthetic agent in the compositions may be in the range of about 10 mg to about 400 mg per unit dose. For example, the amount of lidocaine can be 10 mL of 1% lidocaine per unit dose or 16 mL of 2% lidocaine per unit dose.

The buffer in a composition prepared according to the methods of the present invention can be, but is not limited to, bicarbonate buffer, Tris (Tris(hydroxymethyl)aminomethane) buffer, MOPS buffer (3-(N-morpholino)propanesulfonic acid), HEPES (N-(2-hydroxyethyl)piperazine-N-(2-ethanesulfonic acid) buffer, ACES (2-[(2-amino-2-oxoethyl)amino]ethanesulfonic acid) buffer, ADA (N-(2-acetamido)2-iminodiacetic acid) buffer, AMPSO (3-[(1,1-dimethyl-2-hydroxyethyl)amino]-2-propanesulfonic acid) buffer, BES (N,N-bis(2-hydroxyethyl)-2-aminoethanesulfonic acid buffer, Bicine (N,N-bis(2-hydroxyethylglycine) buffer, Bis-Tris (bis-(2-hydroxyethyl)imino-tris(hydroxymethyl)methane buffer, CAPS (3-(cyclohexylamino)-1-propanesulfonic acid) buffer, CAPSO (3-(cyclohexylamino)-2-hydroxy-1-propanesulfonic acid) buffer, CHES (2-(N-cyclohexylamino)ethanesulfonic acid) buffer, DIPSO (3-[N,N-bis(2-hydroxyethyl)amino]-2-hydroxy-propanesulfonic acid) buffer, HEPPS (N-(2-hydroxyethylpiperazine)-N'-(3-propanesulfonic acid) buffer, HEPPSO (N-(2-hydroxyethyl)piperazine-N'-(2-hydroxypropanesulfonic acid) buffer, MES (2-(N-morpholino)ethanesulfonic acid) buffer, triethanolamine buffer, imidazole buffer, glycine buffer, ethanolamine buffer, phosphate buffer, MOPSO (3-(N-morpholino)-2-hydroxypropanesulfonic acid) buffer, PIPES (piperazine-N,N'-bis(2-ethanesulfonic acid) buffer, POPSO (piperazine-N,N'-bis(2-hydroxypropaneulfonic acid) buffer, TAPS (N-tris[hydroxymethyl)methyl-3-aminopropanesulfonic acid) buffer; TAPSO (3-[N-tris(hydroxymethyl)methylamino]-2-hydroxy-propanesulfonic acid) buffer, TES (N-tris(hydroxymethyl)methyl-2-aminoethanesulfonic acid) buffer, tricine (N-tris(hydroxymethyl)methylglycine buffer), 2-amino-2-methyl-1,3-propanediol buffer, and 2-amino-2-methyl-1-propanol buffer, or a combination thereof. Particularly preferred buffers are bicarbonate buffer and Tris buffer or a combination thereof. A particularly preferred bicarbonate buffer is sodium bicarbonate.

The quantity of buffer in the composition will vary depending on the subject, the severity and course of the disease, the subject's health, the response to treatment, pharmacokinetic considerations such as liver and kidney function, and the judgment of the treating physician. Accordingly, a number of compositions including differing quantities of buffer per unit dose can be prepared by methods according to the present invention. For example, when the buffer is sodium bicarbonate, the amount of sodium bicarbonate may be about 3 mL of 8.4% sodium bicarbonate per unit dose.

A particularly preferred composition prepared by methods according to the present invention can comprise heparin sodium as the heparinoid, lidocaine hydrochloride as the local anesthetic, and sodium bicarbonate as the buffer.

Compositions prepared by methods according to the present invention can include one or more additional optional components. Such additional optional components can include:
(1) an osmolar component that provides an isotonic or nearly isotonic solution compatible with human cells and blood;
(2) a compound that enables persistence of the composition to the surface of the bladder epithelium in a quantity sufficient to treat, ameliorate, or prevent a lower urinary tract disorder;
(3) an antibacterial agent in a quantity sufficient to treat, ameliorate, or prevent a lower urinary tract disorder;
(4) an antifungal agent in a quantity sufficient to treat, ameliorate, or prevent a lower urinary tract disorder;
(5) a vasoconstrictor in a quantity sufficient to treat, ameliorate, or prevent a lower urinary tract disorder; and
(6) a preservative.

When present, the optional osmolar component is a salt, such as sodium chloride, or a sugar or a combination of two or more of these components. The sugar may be a monosaccharide such as dextrose, a disaccharide such as sucrose or lactose, a polysaccharide such as dextran 40, dextran 60, or starch, or a sugar alcohol such as mannitol. It should be obvious to those skilled in the art that all components of the solution contribute to the osmolarity of the solution but to achieve an isotonic or near-isotonic solution, the contributions of those components should be taken into account to ensure that the proper proportion of osmolar component is added and an excess of osmolar component is not added which would result in a hypertonic solution. In fact, when the composition described above including heparin sodium as the heparinoid, lidocaine hydrochloride as the anesthetic, and sodium bicarbonate as the buffer, the osmolar contributions of the sodium ion from the heparin sodium and sodium bicarbonate, the chloride ion from the lidocaine hydrochloride, and the carbonate/bicarbonate ion from the sodium bicarbonate are sufficient not to require an additional osmolar component. However, the solution does not need to be isotonic; it can be hypotonic, isotonic, or hypertonic since the bladder epithelium can tolerate a large range of osmolarity.

If an antibacterial agent is present, the antibacterial agent can be selected from the group consisting of a sulfonamide, a penicillin, a combination of trimethoprim plus sulfamethoxazole, a quinolone, methenamine, nitrofurantoin, a cephalosporin, a carbapenem, an aminoglycoside, a tetracycline, and a macrolide. Suitable sulfonamides include, but are not limited to, sulfanilamide, sulfadiazine, sulfamethoxazole, sulfisoxazole, sulfamethizole, sulfadoxine, and sulfacetamide. Suitable penicillins include, but are not limited to, methicillin, nafcillin, oxacillin, cloxacillin, dicloxacillin, ampicillin, amoxicillin, bacampicillin, carbenicillin, ticarcillin, mezlocillin, and piperacillin. Suitable quinolones include, but are not limited to, nalidixic acid, cinoxacin, norfloxacin, ciprofloxacin, orfloxacin, sparfloxacin, lomefloxacin, fleroxacin, pefloxacin, and amifloxacin. Suitable cephalosporins include, but are not limited to, cephalothin, cephazolin, cephalexin, cefadroxil, cefamandole, cefoxatin, cefaclor, cefuroxime, loracarbef, cefonicid, cefotetan; ceforanide, cefotaxime, cefpodoxime proxetil, ceftizoxime, ceftriaxone, cefoperazone, ceftazidime, and cefepime. Suitable carbepenems include, but are not limited to, imipenem, meropenem, and aztreonam. Suitable aminoglycosides include, but are not limited to, netilmycin and gentamicin. Suitable tetracyclines include, but are not limited to, tetracycline, oxytetracycline, demeclocycline, minocycline, doxycycline, and chlortetracycline. Suitable macrolides include, but are not limited to, erythromycin, clarithromycin, and azithromycin.

If an antifungal agent is present, the antifungal agent can be selected from the group consisting of amphotericin B, itraconazole, ketoconazole, fluconazole, miconazole, and flucytosine.

If a vasoconstrictor is present, the vasoconstrictor can be epinephrine.

If a compound that enables persistence of the composition to the surface of the bladder epithelium is present, the compound is typically an activatable gelling agent. The activatable gelling agent is typically a thermoreversible gelling agent. The thermoreversible gelling agent can be selected from the group consisting of Pluronics F127 gel, Lutrol gel, N-isopropylacrylamide, ethylmethacrylate, N-acryloxysuccinimide, xyloglucan sols of 1-2%, graft copolymers of pluronic and poly(acrylic acid), pluronic-chitosan hydrogels, and a [poly(ethylene glycol)-poly[lactic acid-co-glycolic acid]-poly(ethylene glycol)] (PEG-PLGA-PEG) copolymer.

If a preservative is present, the preservative can be selected from the group consisting of parabens, chlorobutanol, phenol, sorbic acid, thimerosal, and benzoic acid; benzoic acid is now being used clinically with good success. However, typically, compositions prepared by methods according to the present invention do not require a preservative component, as the compositions are prepared and dispensed in sealed single-unit-dose vials.

If any of these optional components, i.e., the osmolar component, the compound that enables persistence of the composition to the surface of the bladder epithelium, the antibacterial component, the antifungal compound, the vasoconstrictor, or the preservative, are present, they are typically added after a stable solution including the heparinoid, the local anesthetic, and the buffer has been prepared. The quantities of these additional optional components, if used, are chosen such that the solution of the heparinoid, the local anesthetic, and the buffer remains stable and precipitation of the local anesthetic is avoided and the final pH of the solution is achieved; the final pH is typically from about 6.7 to about 8.3 as described above An optimum pH is about 7.4.

If sterilization of the composition is required, it is typically performed by filtration. Other sterilization methods are known in the art.

The heparinoid, the local anesthetic, and the buffer are as described above. In particular, a preferred heparinoid is heparin, a preferred local anesthetic is lidocaine, in the form of lidocaine hydrochloride, and a preferred buffer is sodium bicarbonate.

These methods can further comprise an additional step of adding one or more of: (i) an osmolar component; (ii) a compound that enables persistence of the composition to the surface of the bladder epithelium; (iii) an antibacterial agent; (iv) an antifungal agent; (v) a vasoconstrictor; or (vi) a preservative, subsequent to preparation of a buffered composition including the heparinoid, the local anesthetic, and the buffer.

Preferred components for the heparinoid, the local anesthetic, and the buffer are as described above.

Yet another aspect of the present invention is a premixed liquid composition comprising a heparinoid, a local anesthetic, and a buffer prepared according to the method above that is stable for at least three months without precipitation of the local anesthetic. Typically, the composition is prepared by a method according to the present invention as described above. Typically, in this composition, the heparinoid is heparin, the local anesthetic is lidocaine, and the buffer is sodium bicarbonate. Typically, the pH of this composition is from 6.7 to 8.3. Preferably, the pH of this composition is from 7.0 to 7.8. More preferably, the pH of this composition is about 7.4.

Compositions according to the present invention are suitable for treating, ameliorating, or preventing a lower urinary tract disorder selected from the group consisting of bacterial cystitis, fungal/yeast cystitis, vulvar vestibulitis, vulvodynia, dyspareunia, urethral syndrome, and endometriosis in women; prostatitis and chronic pelvic pain syndrome in men; and radiation-induced cystitis, chemotherapy-induced cystitis, interstitial cystitis, and overactive bladder in men or women. Compositions according to the present invention are particularly useful in treating interstitial cystitis.

As used herein, the terms "treat, ameliorate, or prevent" refer to any detectable improvement, whether subjective or objective, in the lower urinary tract disorder of the subject to whom the composition is administered. For example, the terms "treat, ameliorate, or prevent" can refer to an improvement as determined by the PORIS scale, the PUF scale, or any component of those scales; reduction of pain; reduction of urinary frequency; reduction of urinary urgency; reduction of requirement for narcotic administration; reduction of incontinence; reduction of abnormal permeability of the urothelium to potassium; or improvement in more than one of these parameters. The terms "treat, ameliorate, or prevent" do not state or imply a cure for the underlying lower urinary tract disorder.

The invention is illustrated by the following Example. This Example is included for illustrative purposes only.

### EXAMPLE

The following experiment was performed to test the efficacy of compositions prepared according to the present invention and compare them with compositions prepared by previous methods.

Solution 1 was prepared as follows: Commercially available heparin, lidocaine (approved for human use by the FDA) were mixed as follows: 5 ml heparin solution containing 50,000 units of heparin, 5 ml of 4% lidocaine (200 mg) and 2 ml of 8.4% sodium bicarbonate (168 mg) were mixed by adding the heparin and bicarbonate together and then lastly adding lidocaine with a final pH of 7.4. The solution became turbid after 2-3 minutes reflecting loss of lidocaine from solution.

Solution 2 was prepared as follows: Powdered sodium heparin (50,000 units) and powdered lidocaine hydrochloride (200 mg) were dissolved in a total of 13 ml water and 2 ml of 8.4% sodium bicarbonate (168 mg) were was then added, yielding a final pH of 7.4. The solution was sterile filtered and put into a stoppered vial and remained clear and stable. However if the first step was to mix the liquid lidocaine with buffer without the presence of the heparin, the solution became turbid reflecting the loss of lidocaine from solution.

To test clinically the two different solutions (Solutions 1 and 2, above), they were each given to 5 different patients with diagnosed interstitial cystitis. The patients received in a blinded fashion each solution on different days and were then asked to compare the solutions. All five found Solution 2 to be at least 50% more effective than Solution 1.

These results indicate improved efficacy of a composition prepared by a method according to the present invention, with improved bioavailability of the local anesthetic, in this case, lidocaine.

It proved possible to compose the solution and avoid precipitation of the lidocaine at a pH value greater than pH 8.3.

Clinically patients have done well with three pH's tested; one was 7.06, another was 7.45 and yet another was 7.72. In additional at all pH's the lidocaine was in solution when starting with powdered heparin and lidocaine being mixed and then buffered accordingly with either tris or sodium bicarbonate.

### ADVANTAGES OF THE INVENTION

The present invention provides an improved method of preparing compositions for treatment of a lower urinary tract disorder that include a heparinoid, a local anesthetic, and a buffer, typically heparin, lidocaine, and sodium bicarbonate. Compositions prepared by the method of the present invention are stable for three months or more and do not undergo precipitation of the local anesthetic. Accordingly, they retain efficacy and the local anesthetic retains bioavailability, an improvement over previously available compositions.

Compositions according to the present invention possess industrial applicability as compositions intended for medical use, specifically to treat lower urinary tract diseases and conditions. Methods according to the present invention possess industrial applicability for the preparation of a medicament to treat lower urinary tract diseases and conditions.

With respect to ranges of values, the invention encompasses each intervening value between the upper and lower limits of the range to at least a tenth of the lower limit's unit, unless the context clearly indicates otherwise. Moreover, the invention encompasses any other stated intervening values and ranges including either or both of the upper and lower limits of the range, unless specifically excluded from the stated range.

Unless defined otherwise, the meanings of all technical and scientific terms used herein are those commonly understood by one of ordinary skill in the art to which this invention belongs. One of ordinary skill in the art will also appreciate that any methods and materials similar or equivalent to those described herein can also be used to practice or test this invention.

## Claims

1. A method for preparing a composition for treatment of a lower urinary tract condition by instilling the composition into the urinary bladder, the composition comprising a mixture comprising a heparinoid, a local anesthetic, and a buffer, the method comprising the steps of:
(a) providing a heparinoid in solid form;
(b) providing a local anesthetic in solid form;
(c) dissolving the heparinoid in solid form and the local anesthetic in solid form in water;
(d) adding a liquid buffer to the mixture of the heparinoid and the local anesthetic dissolved in water to form a mixture of the liquid buffer, the heparinoid, and the local anesthetic; and
(e) adjusting the pH of the mixture of the liquid buffer, the local anesthetic, and the heparinoid so that a pH of from 6.7 to 8.3 is achieved without precipitation of the local anesthetic.

2. The method of claim 1 wherein the heparinoid is heparin.

3. The method of claim 2 wherein the heparinoid in solid form is heparin sodium.

4. The method of claim 1 wherein the local anesthetic is lidocaine.

5. The method of claim 4 wherein the local anesthetic in solid form is lidocaine hydrochloride.

6. The method of claim 1 wherein the buffer is sodium bicarbonate.

7. The method of claim 1 wherein the buffer is selected from the group consisting of phosphate buffer and Tris buffer.

8. The method of claim 1 wherein the method further comprises the step of adding one or more of: (i) an osmolar component; (ii) a compound that enables persistence of the composition to the surface of the bladder epithelium; (iii) an antibacterial agent; (iv) an antifungal agent; (v) a vasoconstrictor; or (vi) a preservative, subsequent to preparation of a buffered composition including the heparinoid, the local anesthetic, and the buffer.

9. A premixed liquid composition for treatment of a lower urinary tract condition by instilling the composition into the urinary bladder, which is obtained according to a method of any of claims 1 to 8 and comprises a heparinoid, a local anesthetic, and a buffer, wherein the composition is stable for at least three months without precipitation of the local anesthetic.

10. The composition of claim 9 wherein the heparinoid is heparin, the local anesthetic is lidocaine, and the buffer is selected from the group consisting of sodium bicarbonate, phosphate buffer and Tris buffer.

## Patentansprüche

1. Verfahren zur Herstellung einer Zusammensetzung zur Behandlung von Beschwerden der unteren Harnwege, indem die Zusammensetzung in die Harnblase eingeträufelt wird, wobei die Zusammensetzung eine Mischung aufweist, welche ein Heparinoid, ein Lokalanästhetikum und einen Puffer enthält, wobei das Verfahren die folgenden Schritte umfasst:
(a) Bereitstellen eines Heparinoids in fester Form;
(b) Bereitstellen eines Lokalanästhetikums in fester Form;
(c) Auflösen des Heparinoids in fester Form und des Lokalanästhetikums in fester Form in Wasser;
(d) Zugeben eines flüssigen Puffers zu der Mischung aus dem in Wasser gelösten Heparinoid und Lokalanästhetikum zur Bildung einer Mischung aus dem flüssigen Puffer, dem Heparinoid und dem Lokalanästhetikum; sowie
(e) Einstellen des pH der Mischung aus dem flüssigen Puffer, dem Lokalanästhetikum und dem Heparinoid derart, dass ein pH von 6,7 bis 8,3 ohne Ausfällen des Lokalanästhetikums erzielt wird.

2. Verfahren nach Anspruch 1, wobei das Heparinoid aus Heparin besteht.

3. Verfahren nach Anspruch 2, wobei das Heparinoid in fester Form aus Heparin-Natrium besteht.

4. Verfahren nach Anspruch 1, wobei das Lokalanästhetikum aus Lidocain besteht.

5. Verfahren nach Anspruch 4, wobei das Lokalanästhetikum in fester Form aus Lidocainhydrochlorid besteht.

6. Verfahren nach Anspruch 1, wobei der Puffer aus Natriumbicarbonat besteht.

7. Verfahren nach Anspruch 1, wobei der Puffer aus der Gruppe bestehend aus Phosphatpuffer und TRIS-Puffer ausgewählt ist.

8. Verfahren nach Anspruch 1, wobei das Verfahren weiterhin den Schritt des Zugebens eines oder mehr aus: (i) einem osmolaren Bestandteil; (ii) einer Verbindung, die eine Persistenz der Zusammensetzung an der Oberfläche des Blasenepithels ermöglicht; (iii) einem antibakteriellen Wirkstoff; (iv) einem antimykotischen Wirkstoff; (v) einem Vasokonstriktor; oder (vi) einem Konservierungsmittel, nach der Herstellung einer gepufferten Zusammensetzung mit dem Heparinoid, dem Lokalanästhetikum und dem Puffer aufweist.

9. Vorgemischte flüssige Zusammensetzung zur Behandlung von Beschwerden der unteren Harnwege durch Einträufeln der Zusammensetzung in die Harnblase, welche gemäß einem Verfahren nach einem der Ansprüche 1 bis 8 erhalten wird und ein Heparinoid, ein Lokalanästhetikum sowie einen Puffer aufweist, wobei die Zusammensetzung über mindestens drei Monate ohne Ausfällen des Lokalanästhetikums stabil ist.

10. Zusammensetzung nach Anspruch 9, wobei das Heparinoid aus Heparin besteht, das Lokalanästhetikum aus Lidocain besteht und der Puffer aus der Gruppe bestehend aus Natriumbicarbonat, Phosphatpuffer und TRIS-Puffer ausgewählt ist.

## Revendications

1. Procédé de préparation d'une composition destinée au traitement d'un état du bas appareil urinaire en instillant la composition dans la vessie urinaire, la composition comportant un mélange comportant un héparinoïde, un anesthésique local et un tampon, le procédé comportant les étapes consistant à :
(a) fournir un héparinoïde sous forme solide ;
(b) fournir un anesthésique local sous forme solide ;
(c) dissoudre l'héparinoïde sous forme solide et l'anesthésique local sous forme solide dans de l'eau ;
(d) ajouter un tampon liquide au mélange constitué de l'héparinoïde et de l'anesthésique local dissous dans l'eau pour former un mélange du tampon liquide, de l'héparinoïde et de l'anesthésique local ; et
(e) ajuster le pH du mélange du tampon liquide, de l'anesthésique local et de l'héparinoïde de sorte qu'un pH de 6,7 à 8,3 est obtenu sans précipitation de l'anesthésique local.

2. Procédé selon la revendication 1, dans lequel l'héparinoïde est l'héparine.

3. Procédé selon la revendication 2, dans lequel l'héparinoïde sous forme solide est l'héparine sodique.

4. Procédé selon la revendication 1, dans lequel l'anesthésique local est la lidocaïne.

5. Procédé selon la revendication 4, dans lequel l'anesthésique local sous forme solide est l'hydrochlorure de lidocaïne.

6. Procédé selon la revendication 1, dans lequel le tampon est le bicarbonate de sodium.

7. Procédé selon la revendication 1, dans lequel le tampon est choisi parmi le groupe constitué d'un tampon de phosphate et d'un tampon Tris.

8. Procédé selon la revendication 1, dans lequel le procédé comporte en outre l'étape consistant à ajouter un ou plusieurs éléments parmi : (i) un composant osmolaire ; (ii) un composé qui permet la persistance de la composition à la surface de l'épithélium de vessie ; (iii) un agent antibactérien ; (iv) un agent antifongique ; (v) un vasoconstricteur ; ou (vi) un conservateur, après la préparation d'une composition tamponnée incluant l'héparinoïde, l'anesthésique local et le tampon.

9. Composition liquide pré-mélangée pour le traitement d'un état du bas appareil urinaire en instillant la composition dans la vessie urinaire, qui est obtenue selon un procédé de l'une quelconque des revendications 1 à 8 et comporte un héparinoïde, un anesthésique local et un tampon, dans laquelle la composition est stable pendant au moins trois mois sans précipitation de l'anesthésique local.

10. Composition selon la revendication 9, dans laquelle l'héparinoïde est l'héparine, l'anesthésique local est la lidocaïne, et le tampon est choisi parmi le groupe constitué du bicarbonate de sodium, d'un tampon de phosphate et d'un tampon Tris.
